# EUROPEAN PATENT SPECIFICATION

(11) **EP 3 946 313 B1**
(45) Date of publication and mention of the grant of the patent: **27.05.2026**
(21) Application number: 20779652.5
(22) Date of filing: 25.03.2020
(51) Int. Cl.: A61K 31/58, A61K 31/519, A61K 45/06, A61P 35/00, A61K 9/00

(54) **ONVANSERTIB FOR THE TREATMENT OF METASTATIC CASTRATION-RESISTANT PROSTATE CANCER**
ONVANSERTIB ZUR BEHANDLUNG VON METASTASIERTEM, KASTRATIONSRESISTENTEM PROSTATAKREBS
ONVANSERTIB POUR LE TRAITEMENT DU CANCER DE LA PROSTATE MÉTASTATIQUE RÉSISTANT À LA CASTRATION

(30) Priority: 28.03.2019 US 201962825634 P; 22.08.2019 US 201962890209 P
(43) Date of publication of application: 09.02.2022
(73) Proprietor: Cardiff Oncology, Inc., San Diego CA 92121 (US)
(72) Inventor: ERLANDER, Mark, San Diego, CA 92121 (US); ADAMS, Thomas, H., San Diego, CA 92121 (US); RIDINGER, Maya, San Diego, CA 92121 (US)
(74) Representative: J A Kemp LLP
(86) International application number: PCT/US2020/024572
(87) International publication number: WO 2020/198281

(56) References cited:
- WO-A2-2017/053763
- US-A1- 2015 209 359
- ANONYMOUS: "NCT03414034: Onvansertib in Combination With Abiraterone and Prednisone in Adult Patients With Metastatic Castration-Resistant Prostate Cancer", 29 January 2018 (2018-01-29), XP055972789, Retrieved from the Internet <URL:https://clinicaltrials.gov/ct2/show/NCT03414034> [retrieved on 20221019]
- KRASNOV G S ET AL: "Molecular genetic mechanisms of drug resistance in prostate cancer", MOLECULAR BIOLOGY : COVER-TO-COVER TRANSLATION = MOLEKULYARNAYA BIOLOGIYA, ACADEMY OF SCIENCES OF THE USSR, RU, vol. 49, no. 5, 10 October 2015 (2015-10-10), pages 638 - 648, XP035554856, ISSN: 0026-8933, [retrieved on 20151010], DOI: 10.1134/S0026893315050118
- "Trovagene Receives USAN Approval for ''Onvansertib'' as Nonproprietary Name for First-in-Class, 3rd Generation PLK1 Inhibitor Drug Candidate, PCM-075", PRNEWSWIRE, 15 August 2018 (2018-08-15), pages 1 - 5, XP055744436
- DUTT, SS ET AL.: "Molecular mechanisms of castration-resistant prostate cancer progression", FUTURE ONCOLOGY, vol. 5, November 2009 (2009-11-01), pages 1403 - 1413, XP055744438
- EINSTEIN DAVID JOHNSON, CHOUDHURY ATISH DIPANKAR, SAYLOR PHILIP JAMES, WERNER LILLIAN, ERLANDER MARK G., RIDINGER MAYA, BUBLEY GLE: "A phase II study of onvansertib in combination with abiraterone and prednisone in patients with metastatic castration-resistant prostate cancer (mCRPC", JOURNAL OF CLINICAL ONCOLOGY, vol. 38, no. 6_suppl, 19 February 2020 (2020-02-19), US, pages TPS266 - TPS266, XP009530161, ISSN: 0732-183X, DOI: 10.1200/JCO.2020.38.6_suppl.TPS266

## Description

### BACKGROUND OF THE INVENTION

### (1) Field of the Invention

The present application generally relates to treatments for prostate cancer. More specifically, the application is directed to the treatment of prostate cancer with PLK1 inhibitors to stabilize PSA levels in patients treated with an antiandrogen or an androgen antagonist.

### (2) Description of the related art

### Metastatic Castration-Resistant Prostate Cancer

Prostate cancer (PCa) is the second most frequently diagnosed cancer and fifth most common cause of cancer death among men, causing an estimated 300,000 deaths worldwide in 2012 [WHO, 2014]. Although most men with metastatic prostate cancer initially respond to the historical standard-of-care, androgen-deprivation therapy, resistance inevitably develops after months to years, which is then known as metastatic castration-resistant prostate cancer (mCRPC). Resistance may be mediated by reactivation of androgen-receptor signaling through persistent adrenal androgen production, up-regulation of intratumoral testosterone production, modification of the biologic characteristics of androgen receptors, and steroidogenic parallel pathways [Yamaoka et al., 2010; Sharp et al., 2019; Armstrong and Lao, 2018; Cao et al., 2016; Vlachostergios et al., 2017; Wadosky and Koochekpour, 2017]. Despite the availability of multiple hormonal and non-hormonal agents, survival after the diagnosis of mCRPC remains limited. In addition, targeted approaches based on biomarkers have only recently emerged in this setting [Mateo et al., 2015].

Abiraterone acetate is the prodrug of abiraterone. It targets cytochrome P-450c17, a critical enzyme in androgen biosynthesis. The active D4A metabolite inhibits multiple steroidogenic enzymes and antagonizes the androgen receptor [Li, et al., 2015]. Large randomized trials have demonstrated an overall survival benefit with abiraterone for mCRPC before [de Bono et al., 2011; Fizazi et al., 2012] or after [Ryan et al., 2013] docetaxel therapy. It may also be effective in the neoadjuvant setting for localized PCa [Taplin et al., 2014], although phase 3 trials are ongoing. Finally, two recent studies demonstrated a survival advantage of early use of abiraterone in metastatic castration-sensitive prostate cancer (mCSPC) [Taplin et al., 2014; Fizazi et al., 2017], as opposed to its use at the time of castration-resistance as second-line therapy. Therefore, the standard of care has recently changed to include first-line use of abiraterone in combination with androgen-deprivation therapy.

Although abiraterone is clearly effective in both mCSPC and mCRPC, patients will still inevitably develop resistance. In addition, the efficacy of subsequent hormonal manipulations after abiraterone is often limited [de Bono and Spears, 2017]. Thus, abiraterone-resistant PCa is still a major clinical challenge. As abiraterone is increasingly used in first-line setting, a growing population of patients will eventually experience secondary disease progression that is resistant to further hormonal manipulation [Fizazi et al., 2017]. At this point, only docetaxel, cabazitaxel, and radium-223 have demonstrated survival benefits in trials [Chi et al., 2017; Tannock et al., 2004; Petrylak et al., 2004; de Bono et al., 2010], but only on the order of months versus comparator therapies; median overall survival in all trials was limited to less than two years. Benefits may be even more limited in the modern patient population that has had access to abiraterone and an androgen receptor antagonist such asenzalutamide. Clearly, new and better therapeutic options for PCa are urgently needed.

### Polo-like Kinase 1

Polo-like kinase 1 (PLK1) is the most well characterized member of the 5 members of the family of serine/threonine protein kinases and strongly promotes the progression of cells through mitosis. PLK1 performs several important functions throughout mitotic (M) phase of the cell cycle, including the regulation of centrosome maturation and spindle assembly, the removal of cohesins from chromosome arms, the inactivation of anaphase-promoting complex/cyclosome inhibitors, and the regulation of mitotic exit and cytokinesis [Parker et al., 2013]. During the various stages of mitosis PLK1 localizes to the centrosomes, kinetochores and central spindle.

PLK1 is ubiquitously expressed in normal proliferating tissues and is over-expressed in a wide variety of human tumors (including lung, colon, prostate, ovary, breast, head and neck squamous cell carcinoma [Wolf et al., 1997; Weichert et al., 2004(a), 2004(b), 2005(a), 2005(b); Knect et al., 1999]), and hematologic malignancies [Renner et al., 2009; Mito et al., 2005; Ikezoe et al., 2009]. In addition, several studies have shown that this over expression correlates with poor prognosis. For example, in head and neck squamous cell carcinoma, the 5-year survival rate of patients with medium vs high expression levels falls from 43% to 12% in patients with tumors over-expressing PLK1 [Knect et al., 1999].

In addition, PLK1 is not expressed in differentiated postmitotic cells such as neurons, where instead, expression of PLK2 and PLK3 are detected [Kauselmann et al., 1999]. Therefore, PLK1 selective inhibitors could have an advantage in comparison with classical antimitotic agents like taxanes or vinca alkaloids, which cause major side effects such as neuropathy, as PLK1 selective inhibitors do not act on tubulins that are present in non-proliferating tissues (such as neurons) [Jackson et al., 2007]. US 2015/209359 describes the use of PLK1 inhibitors such as BI2356 for use in the treatment of prostate cancer. These properties make PLK1 a very attractive new target for cancer therapy [Strebhardt and Ullrich, 2006].

### Onvansertib

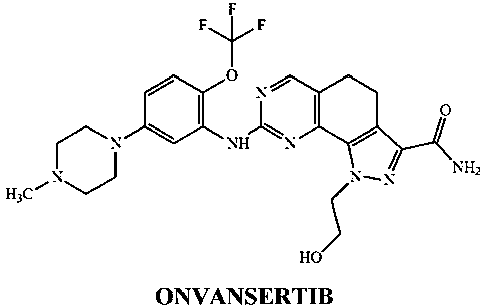

Onvansertib (also known as PCM-075, NMS-1286937, NMS-937, "compound of formula (I)" in U.S. Patent 8,927,530; IUPAC name 1-(2-hydroxyethyl)-8-{[5-(4-methylpiperazin-1-yl)-2-(trifluoromethoxy) phenyl] amino}- 4,5-dihydro-1H-pyrazolo[4,3-h] quinazoline-3-carboxamide) is the first PLK1 specific adenosine triphosphate competitive inhibitor administered by oral route to enter clinical trials with proven antitumor activity in different preclinical models [Weiss et al., 2017]. For example, Clinical Trial NCT03414034 (see also J. Clin. Oncol. 2020, 36(6-suppl), TPS266) relates to a phase II study of onvansertib in combination with abiraterone and prednisone in patients with metastatic castration-resistant prostate cancer. The compound shows high potency in proliferation assays having low nanomolar activity on a large number of cell lines, both from solid as well as hematologic tumors. Onvansertib potently causes a mitotic cell-cycle arrest followed by apoptosis in cancer cell lines and inhibits xenograft tumor growth with a clear PLK1-related mechanism of action at well tolerated doses in mice after oral administration. Onvansertib has favorable pharmacologic parameters and good oral bioavailability in rodent and nonrodent species, as well as proven antitumor activity in different nonclinical models using a variety of dosing regimens, which may potentially provide a high degree of flexibility in dosing schedules, warranting investigation in clinical settings.

The major metabolic pathways found in the different animal species were N-oxidation of the N methyl-piperazine ring to give N-oxide M2 and hydroxylation on an aliphatic carbon atom of the methylene bridge of the pyrazoloquinazoline moiety to give metabolite M1. Qualitatively, no marked differences in the metabolism of onvansertib were observed between species and, quantitatively, some differences were observed cross-species.

Onvansertib has been evaluated pre-clinically in combination with more than 10 different chemotherapeutics, including cisplatin, cytarabine, doxorubicin, gemcitabine and paclitaxel, as well as targeted therapies such as abiraterone, HDAC inhibitors, FLT3 inhibitors, and bortezomib. These therapeutics are used clinically for treatment of many hematologic and solid cancers, including acute myeloid leukemia, non-Hodgkin's lymphoma, metastatic castration-resistant prostate cancer, adrenocortical carcinoma, triple negative breast cancer, small cell lung cancer, and ovarian cancer.

A Phase 1 safety study with onvansertib has been conducted in adult patients with advanced/metastatic solid tumors at a single study site in the U.S. [Weiss et al., 2017]. The primary objective was to determine first cycle dose-limiting toxicities (DLT) and maximum tolerated dose (MTD) of onvansertib administered orally for 5 consecutive days every 3 weeks (ie, 21-day treatment cycle). Secondary objectives were to define the safety profile of onvansertib, determine the pharmacokinetics (PK) of onvansertib in plasma (at the MTD), and document any antitumor activity.

A total of 21 patients were enrolled, and 19 patients were treated. No DLTs occurred at the first 3 dose levels (6, 12, and 24 mg/m²/day). At the subsequent dose level (48 mg/m²/day), 2 of 3 patients developed DLTs. An intermediate level of 36 mg/m²/day was therefore investigated. Four patients were treated and two DLTs were observed. After further cohort expansion, the MTD was determined to be 24 mg/m²/day.

The best observed treatment response was disease stabilization, which occurred in 5 of the 16 evaluable patients. One patient among the 16 evaluable patients had prostate cancer and had disease progression. The study identified thrombocytopenia and neutropenia as the primary toxicities, which is consistent with the expected mechanism of action of onvansertib and results from preclinical studies. These hematologic toxicities were reversible, with recovery usually occurring within 3 weeks.

There is an urgent unmet medical need for patients who have prostate cancer, in particular mCRPC. PLK1 is one of the most upregulated pathways in prostate cancer following castration [Li et al., 2015]. Loss of phosphatase and tensin homolog deleted on chromosome 10 (PTEN) tumor suppressor is a majority driver of advanced prostate cancer but results in mitotic stress. Inactivation of PTEN is correlated with overexpression of PLK1, which is critical for PTEN-depleted cells to adapt to mitotic stress. [Liu et al., 2011]. The mechanism may be a result of the regulatory effect of nuclear PTEN on the E3 ubiquitin ligase anaphase-promoting complex/cyclosome APC-Cdh1, which ubiquitinates and degrades PLK1 [Song et al., 2011]. PLK1 confers tumorigenic competence of PTEN-depleted prostate cancer cells in a mouse xenograft model, and inhibition by a PLK1 kinase inhibitor or siRNA preferentially suppresses tumor growth of PTEN-depleted cells [Liu et al., 2011]. Preclinical evidence from both in-vitro and in-vivo studies indicate that PLK1 inhibition may enhance the efficacy of abiraterone in PCa [Zhang et al., 2014; Zhang et al., 2015]. Multiple mechanisms for the observed synergy of PLK1 inhibition plus abiraterone have been proposed. Liu and colleagues have observed that oxidative stress activated the PI3K-AKT-mTOR pathway and androgen receptor (AR) signaling in a PLK1-dependent manner in prostate cancer cells. In addition, Plk1 inhibition down-regulated SREBP-dependent expression of enzymes involved in androgen biosynthesis. Finally, PLK1 inhibition enhanced cellular responses to abiraterone and overcame abiraterone resistance in cultured PCa cells and patient-derived tumor xenografts [Zhang et al., 2014; Zhang et al., 2015].

### BRIEF SUMMARY OF THE INVENTION

The invention provides onvansertib for use in a method of treating metastatic castration resistant prostate cancer (mCRPC) in a patient, wherein the patient has mCRPC having an altered androgen receptor AR-V7, the method comprising treating the patient with onvansertib in combination with abiraterone and prednisone.

### BRIEF DESCRIPTION OF THE SEVERAL VIEWS OF THE DRAWINGS

FIG. 1 is graphs showing prostate specific antigen (PSA) levels in four prostate cancer patients being treated with abiraterone, prednisone and onvansertib.
FIG. 2 is graphs showing predicted concentrations of onvansertib during onvansertib treatment cycles.
FIG. 3 is a graph showing absolute neutrophil counts during multiple onvansertib treatment cycles.
FIG. 4 is a graph showing PSA levels prior to, and during treatment of prostate cancer patient 03-013 with onvansertib under the Arm A dosing schedule.
FIG. 5 is a graph showing changes in PLA levels of three prostate cancer patients undergoing the Arm B dosing schedule.
FIG. 6A is a graph showing PSA levels prior to, and during treatment of prostate cancer patient 01-024 with onvansertib under the Arm B dosing schedule.
FIG. 6B is a graph showing PSA levels prior to, and during treatment of prostate cancer patient 01-024 with onvansertib under the Arm B dosing schedule.
FIG. 7 is a graph showing outcomes of multiple trial patients in Arm A and Arm B.
FIG. 8 is a graph showing percentage change in circulating tumor cells (CTC) at 12 weeks from baseline in several trial patients.
FIG. 9 is a graph showing percentage change in PSA levels in trial patients.

### DETAILED DESCRIPTION

The invention is defined by the claims. Any subject-matter falling outside the scope of the claims is provided for information purposes only. Any references in the description to methods of treatment, diagnosis or surgery refer to the compounds, pharmaceutical compositions and medicaments of the present invention for use in a method for treatment, diagnosis or surgery of the human or animal body by therapy.

The present invention is based in part on the discovery that PLK1 inhibitors, in particular onvansertib, can stabilize prostate specific antigen (PSA) levels, particularly in patients being treated with an antiandrogen or an androgen antagonist (see Examples below) and in patients having a cancer with an altered androgen receptor that does not require ligand for activation. All references herein to methods of treatment of the human of animal body using a PLK1 inhibitor should be interpreted as meaning the PLK1 inhibitor for use in those methods.

Thus, provided are methods comprising recommending treatment of a prostate cancer patient with a polo-like kinase-1 (PLK1) inhibitor if the patient has rising prostate specific antigen (PSA) levels. In some embodiments, the patient is also treated with the PLK1 inhibitor.

Also provided is a method comprising
measuring prostate specific antigen (PSA) levels in at least two samples from a prostate cancer patient, the samples obtained from the patient at different times; and
recommending treatment of the patient with a PLK1 inhibitor if the PSA levels in the samples increase over time, or not recommending treatment of the patient with a PLK1 inhibitor if the PSA levels in the samples do not increase over time. Some embodiments of these methods further comprise treating the patient with a PLK1 inhibitor if the PSA levels in the samples increase over time, not treating of the patient with a PLK1 inhibitor if the PSA levels in the samples do not increase over time. In some of these embodiments, the patient is also treated with the PLK1 inhibitor.

Additionally provided is a method comprising recommending treatment of a PLK1 inhibitor to a patient having a prostate cancer that has an altered androgen receptor that does not require ligand for activation. In some of these embodiments, the patient is also treated with the PLK1 inhibitor.

Further provided is method comprising recommending treatment with a PLK1 inhibitor to a prostate cancer patient that is being treated with an androgen antagonist and has rising PSA levels. In some of these embodiments, treatment with abiraterone in combination with the PLK1 inhibitor is recommended. In other embodiments, the treatment with the androgen antagonist is discontinued and the patient commences treatment with the PLK1 inhibitor and, optionally, abiraterone.

In these methods, rising PSA levels can be measured in any appropriate manner. Although PSA is normally measured in blood at the present time, the present invention is not limited to rising PSA levels in any particular patient tissue or fluid. See, e.g., Seratec 2011. The PSA tests can be supplemented with other tests that are used to diagnose prostate cancer or evaluate characteristics of the prostate cancer, including but not limited to biopsy and histological examination, PET/CT, PCA3 mRNA, determination of circulating tumor cells, or any other test now known or later discovered. See, e.g., Szeliski et al., 2018.

Rising PSA levels can be determined by evaluating whether there is an increase in PSA level from an earlier to a later patient sample from two or more temporally separated patient samples.

In some embodiments, rising PSA levels are determined by two rising PSA values, separated by a length of time of one month or less, e.g., one day, three days, five days, one week, two weeks, three weeks, four weeks, or any time interval in between.

In various embodiments, the evaluation of PSA level increase from two or more samples also includes at least one confirmatory PSA determination where the rising PSA level does not show a decline.

The amount of rise in PSA levels between the at least two temporally separated samples that is necessary to establish that the PSA levels are rising can be any appropriate amount, e.g., 0.1 ng/mL, 0.2 ng/mL, 0.3 ng/mL, 0.5 ng/mL, 1.0 ng/mL, 3 ng/mL, 5 ng/mL, 10 ng/ml, or any value in between.

In certain specific embodiments, the rising PSA levels are two rising PSA values separated by at least 1 week, one showing a rise of at least 0.3 ng/mL and one confirmatory value not showing a decline.

The efficacy of the PLK1 inhibitor treatment for any patient can be determined by any appropriate method, e.g., any prostate cancer test described above. In some embodiments, a stabilization of PSA levels that would be expected to rise without the PLK1 treatment is used to determine the efficacy of the PLK1 treatment. In some of those embodiments, the efficacious PLK1 inhibitor treatment maintains PSA levels to less than 50%, or 40%, or 30%, or 25%, or 20%, or 15% or 10% or 5% or 0%, or -10%, or -50%, or any lower or in between percentage, above the PSA levels at the start of PLK1 inhibitor treatment.

In some embodiments, the patient has rising PSA levels while being treated with an antiandrogen or an androgen antagonist, with or without prednisone. These embodiments are not limited to any particular antiandrogen or androgen antagonist. In some of these embodiments, the antiandrogen or an androgen antagonist is abiraterone, TOK-001, ARN 509, enzalutamide, apalutibide, darolutamide, or any combination thereof.

According to the invention, the PLK1 inhibitor treatment is provided in combination with abiraterone and prednisone. The PLK1 inhibitor treatment can also be provided in combination with any other treatment, where the other treatment is administered before, after or along with the PLK1 inhibitor treatment. In addition to, abiraterone and prednisone treatment, the patient can be treated with, e.g. any other chemotherapy, radiation, etc.

According to the invention, the PLK1 inhibitor is onvansertib.

The onvansertib may be administered to the patient at any appropriate dosage, e.g., a dosage of less than 12 mg/m², less than or equal to 24 mg/m², or greater than 24 mg/m².

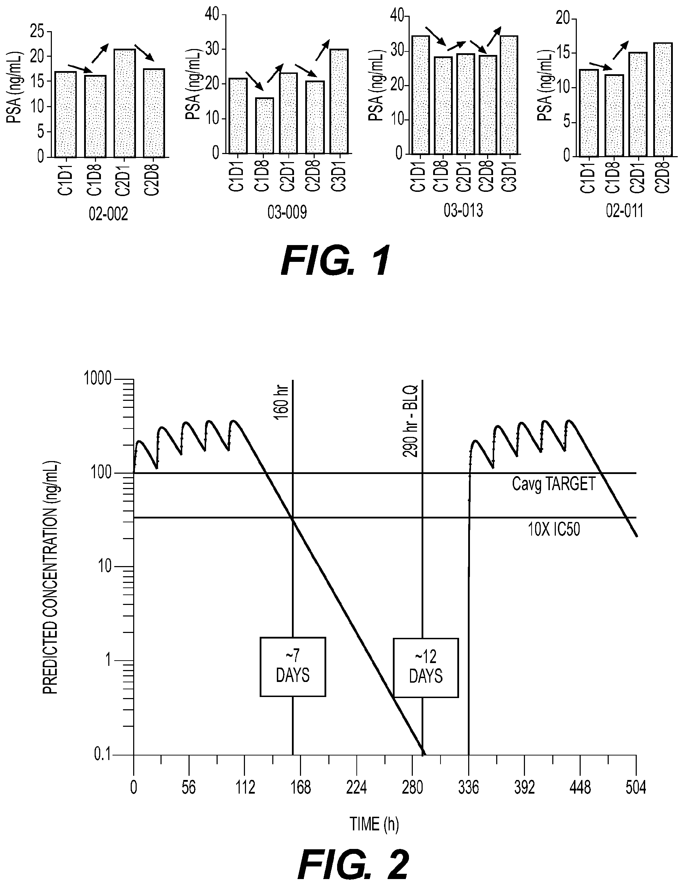

These methods encompass any PLK1 inhibitor dosing schedule. In some of these embodiments, the PLK1 inhibitor is administered to the patient daily. Other nonlimiting examples of dosing schedules within the scope of the methods provided herein include dosing schedules where: the PLK1 inhibitor is administered to the patient in more than one administration cycle where there is 9, 7 or 5 days or less between the administration cycles when no PLK1 inhibitor is administered (see discussion in Example 2 and illustrated in FIG. 2, indicating that more than 9 days after treatment with onvansertib, there is no appreciable amount of onvansertib present); the PLK1 inhibitor is administered in a more than one cycle of 1-10 days of daily administration with 5-16 days with no administration; the PLK1 inhibitor is administered in more than one cycle of 3-7 days of daily administration with 10-16 days with no administration; the PLK1 inhibitor is administered in more than one cycle of 4-6 days of daily administration with 10-16 days with no administration; the PLK1 inhibitor is administered in more than one cycle of 5 days of daily administration with 10-16 days with no administration; the PLK1 inhibitor is administered in more than one cycle of 3-7 days of daily administration with 3-10 days with no administration; the PLK1 inhibitor is administered in more than one cycle of 4-6 days of daily administration with 4-9 days with no administration; the PLK1 inhibitor is administered in more than one cycle of 5 days of daily administration with 5-9 days with no administration; the PLK1 inhibitor is administered in more than one cycle of 2-5 days of daily administration with 5-9 days with no administration; the PLK1 inhibitor is administered in more than one cycle of 2-3 days of daily administration with 5-7 days with no administration; the PLK1 inhibitor is administered in more than one cycle of 2 days of daily administration with 5-6 days with no administration; the PLK1 inhibitor is administered in more than one cycle of 1-2 days of daily administration with 3-8 days with no administration; or the PLK1 inhibitor is administered in more than one cycle of 1 day of daily administration with 5-7 days with no administration.

The methods are useful with a patient with any form of prostate cancer. According to the invention, the prostate cancer is metastatic castration resistant prostate cancer (CRPC).

In some embodiments, the methods also comprise evaluating the prostate cancer to identify androgen receptor variants. In some of these embodiments, the prostate cancer is evaluated by evaluating biopsy tissue. In other embodiments, the prostate cancer is evaluated by evaluating circulating tumor cells (CTC). In additional embodiments, cell-free nucleic acids or protein, e.g., in plasma from the patient, is evaluated to identify androgen receptor variants.

CRPC may be characterized by reactivation of androgen receptor signaling through persistent adrenal androgen production, up-regulation of intratumoral testosterone production, modification of the biologic characteristics of androgen receptors, or steroidogenic parallel pathways. According to the invention, the CRPC is characterized by an altered androgen receptor which is AR-V7. AR-V7 does not require ligand for activation.

Preferred embodiments are described in the following examples. Other embodiments within the scope of the claims herein will be apparent to one skilled in the art from consideration of the specification or practice of the invention as disclosed herein.

### Example 1. Inhibition of human cytochrome P450s with onvansertib

The potential inhibitory capacity of onvansertib towards the major human cytochrome P450 (CYP) isoforms responsible for hepatic drug metabolism in man (CYP1A2, CYP2C8, CYP2C9, CYP2C19, CYP2D6, and CYP3A4) was investigated using human liver microsomes. Results are shown in Table 1. Onvansertib was able to inhibit the metabolic activities of CYP2C8, CYP2C9, CYP2C19, CYP2D6, and CYP3A4 isoforms to different extents, with IC₅₀ values ranging from 20 µM to 66 µM. No significant inhibitory effects against CYP1A2 were found. Considering that the concentrations relevant to achieve significant anti-tumoral activity of the compound in mice were in the order of 1 µM, the likelihood that onvansertib would show clinically relevant metabolic drug-drug interactions is considered low.

**Table 1. Summary of Mean Inhibitor Potency of Onvansertib for Human Liver P450s**

| **P450 Enzyme** | **Enzyme Reaction** | **IC₅₀ (µM)^{a}** |
|---|---|---|
| CYP1A2 | Tacrine 1-hydroxylation | > 100 |
| CYP2C8 | Paclitaxel 6-hydroxylation | 20.2 ± 1.6 |
| CYP2C9 | Diclofenac 4-hydroxylation | 20.4 ± 3.2 |
| CYP2C19 | Mephenytoin 4-hydroxylation | 36.9 ± 15.7 |
| CYP2D6 | Bufuralol 1-hydroxylation | 26.8 ± 5.4 |
| CYP3A4 | Testosterone 6β-hydroxylation | 52.7 ± 9.8 |
| CYP3A4 | Midazolam 1'-hydroxylation | 66.2 ± 4.0 |

| | | |
|---|---|---|
| Source: Report No. 0204-2007-R Abbreviations: IC50=inhibitory drug concentration that produces 50% of the maximal effect; SEM=standard error of mean ^{a}Data are mean±SEM. | | |

### Example 2. Clinical trial

A Phase 2 study, having IND Number 105112, was commenced in 2018 to evaluate the effect of onvansertib in combination with abiraterone and prednisone in patients with metastatic castration-resistant prostate cancer (mCRPC). The inhibition of human cytochrome P450s with onvansertib was also evaluated.

The aim of this ongoing study is to explore treatment with onvansertib in combination with standard of care abiraterone and prednisone in patients with mCRPC. The onvansertib starting dose was 24 mg/m² based on results from the prior Phase 1 trial (Study PLKA-937-001).

### Stabilization of PSA levels by onvansertib

The aim of this ongoing study (IND No. 105112) is to explore treatment with onvansertib in combination with standard of care abiraterone and prednisone in patients with mCRPC. The onvansertib starting dose was 24 mg/m² based on results from the prior Phase 1 trial (Study PLKA-937-001).

The patient treatments are divided into three arms (all arms include daily abiraterone):
- Arm A: onvansertib 24 mg/m² on days 1-5 of a 3 week dosing cycle
- Arm B: onvansertib 18 mg/m² on days 1-5 of a 2 week dosing cycle
- Arm C: onvansertib 12 mg/m² on days 1-14 of a 3 week dosing cycle

The rationale for the addition of Arm B and Arm C (having a shortened number of days between onvansertib treatment) is based on observed temporal changes in PSA values from patients already enrolled in Arm A. Specifically, in many of the patients who have received >1 cycle, changes in PSA appear to be correlated with the dosing schedule. As shown in FIG. 1, after onvansertib dosing on days 1-5, PSA values go down at day 8 relative to baseline but then increase between Day 8 and the start of the next cycle. In particular, for patients 02-002, 03-009 and 03-013, this pattern is observed for both cycles 1 and 2. This may indicate that in a 21-day cycle, the 16 days off enables the tumor to recover and continue to grow (as per observed increased PSA levels).

Pharmacokinetic data from the prior phase 1 trial (at the 24 mg/m² dose) indicates that at 160 hrs (~7 days), the amount of onvansertib is below the 10 X IC₅₀ value for onvansertib, suggesting that patients are receiving a therapeutic level of onvansertib only for days 1-7 of the 21-day cycle. This is consistent with the observed downward changes of PSA values for patients shown above. At 290 hours (~12 days), the drug concentrations are below 0.1 ng/ml, which is the limit of quantitation (BLQ) for assaying onvansertib. Given that the cycle length is 14-days, this suggests that for 3 days there is no appreciable amount of onvansertib present (FIG. 2).

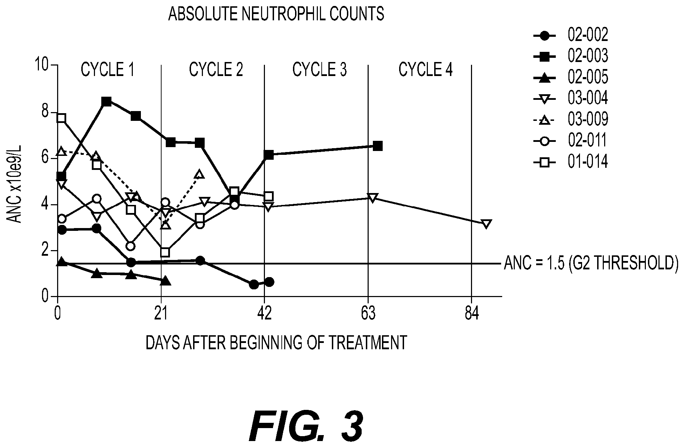

To evaluate this further, neutrophil levels were assessed for patients within Arm A of the current trial. Absolute neutrophil counts (ANC) are plotted in FIG. 3 for 7 patients, with ANC 1.5 indicated as the threshold for Grade 2 (CTCAE version 4.03). Two patients experienced ≥ Grade 2 neutropenia after the first cycle of treatment (02-002 and 02-005). Noteworthy, patient 02-002 had a history of neutropenia with prior Docetaxel treatment, and patient 02-005 had an ANC of 1.54 at baseline (Grade 1 neutropenia).

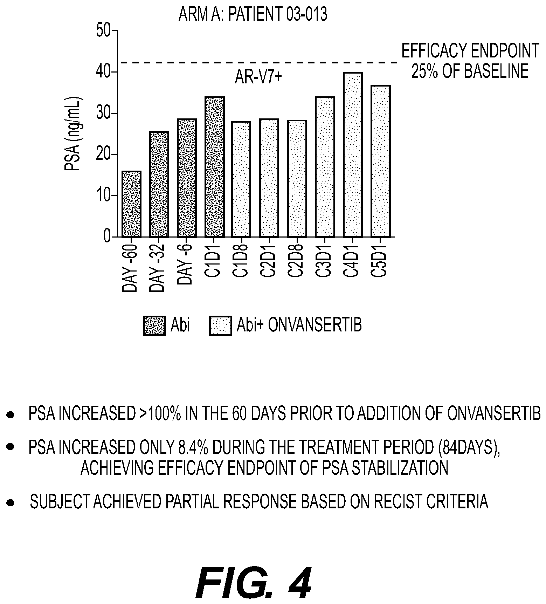

As shown in FIG. 4, Data for further cycles of Arm A treatment for Patient 03-013 shows stabilization of PSA levels into the fifth cycle to below 25% above the baseline PSA level (dotted line), at Cycle 1, Day 1 (C1D1) of onvansertib treatment, prior to onvansertib dosing. Specifically, PSA levels doubled in 60 days while on abiraterone/prednisone prior to onvansertib treatment, but increased only 8.4% after onvansertib was added (84 days), demonstrating disease stabilization. A CT scan at the end of the study indicated ~30% tumor shrinkage. The tumor was assessed at C1D1 to have the AR-V7 androgen receptor variant, which is considered an aggressive tumor that is resistant to anti-androgen therapy.

The significance of the result with Patient 03-013 is underscored by the ability of onvansertib to maintain PSA levels even when those levels rapidly increased on Zytiga (abiraterone) with prednisone alone. The clinical significance of the maintained PSA levels is confirmed by tumor shrinkage, particularly where the tumor type present, with the AR-V7 androgen receptor variant, is known to be resistant to anti-androgen therapy. Additionally, since Patient 03-013 was in Arm A, there were at least two-day periods at the end of each cycle where there was not therapeutic levels of onvansertib. Therefore, the Arms B and C regimen, where the two-day nontherapeutic levels are eliminated at the end of the cycle, is expected to be even more effective.

### Evaluating potential biomarkers of response in circulating tumor cells (CTCs) and circulating tumor DNA (ctDNA)

Several studies have interrogated whether the presence of the constitutively active androgen-receptor splice variant 7 (AR-V7) in tumor cells confers a primary or an acquired resistance to novel androgen receptor signaling inhibitors (ARSi) or other therapies, and whether it could be used as a treatment selection tool in clinical practice. Published data consistently demonstrate that the benefit of ARSi occurs predominantly in AR-V7-negative CRPC patients while most AR-V7-positive CRPC patients do not respond well or durably to abiraterone. We evaluated circulating tumor cells (CTC) for the presence of AR-V7, and ctDNA (Guardant) to determine genomic alterations, from patients in this trial of onvansertib + abiraterone to assess the AR-V7 status.

Results are shown in Table 2. To date, 2 of 6 patients who have completed 4 cycles (12 weeks) of treatment are confirmed AR-V7+ and have shown a PSA response when onvansertib is added to abiraterone, and also had the lowest increase in CTC count from C1D1 to C5D1.

**Table 2. Evaluation of CTCs and ctDNA in study patients**

| **Patient** | **CTC Count (C1D1)** | **CTC Count (C5D1)** | **CTC Fold Change** | **AR-V7 (C1D1)** | **AR-V7 (C5D1)** | **CT-DNA Deleterious Mutation (Gene)** | **CT-DNA Deleterious Mutation (Change)** | **CT-DNA Somatic CNV (Gene)** | **CT-DNA Somatic CNV (Copy Number)** |
|---|---|---|---|---|---|---|---|---|---|
| 02-003 | 4.4 | 78.6 | 17.9 | Negative | Positive | TP53 | p.C275Y_c.824G>A | None | None |
| 03-004 | 0.4 | 1.2 | 3.0 | Negative | Negative | AR | p.T878A_c.2632A>G | AR | 1.72 |
| 02-007 | 2.5 | 17.3 | 6.9 | Negative | Negative | EGFR | p.A822T_c.2464G>A | None | None |
| | | | | | | IDH1 | p.R132C_c.394C>T | | |
| 03-009 | 3.7 | 5.4 | 1.5 | Positive | Negative | BRAF | p.G469A_c.1406G>C | AR | 8.27 |
| | | | | | | | p.R333C_c.997C>T | | |
| | | | | | | STK11 | c.376-2A>C (splice acceptor) | KRAS | 5.92 |
| | | | | | | TP53 | | | |
| 03-013 | 2.2 | 3.0 | 1.4 | Positive | Positive | NF1 | p.11605V_c.4813A>G | CDK6 | 2.32 |
| | | | | | | MYC | p S245Y_c.734C>A | | |
| 01-014 | 87.1 | Not Avail. | N/A | Negative | Not Avail. | Not Tested | Not Tested | Not Tested | Not Tested |

### Example 3. Additional clinical trial results

Additional results from the clinical trial described in Example 2 are provided herewith.

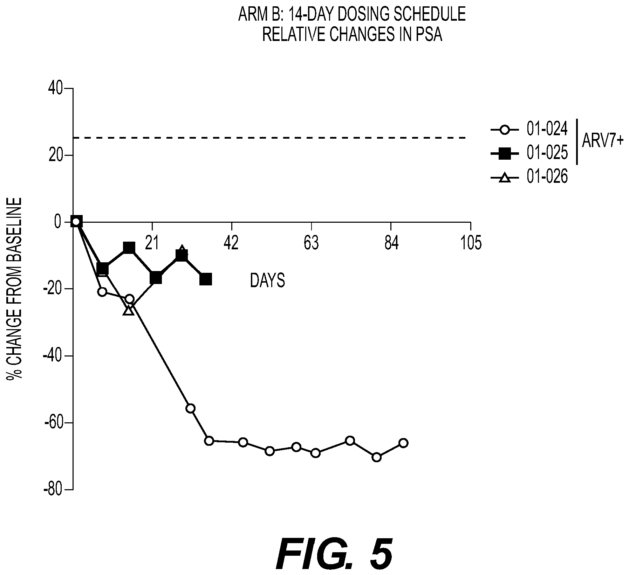

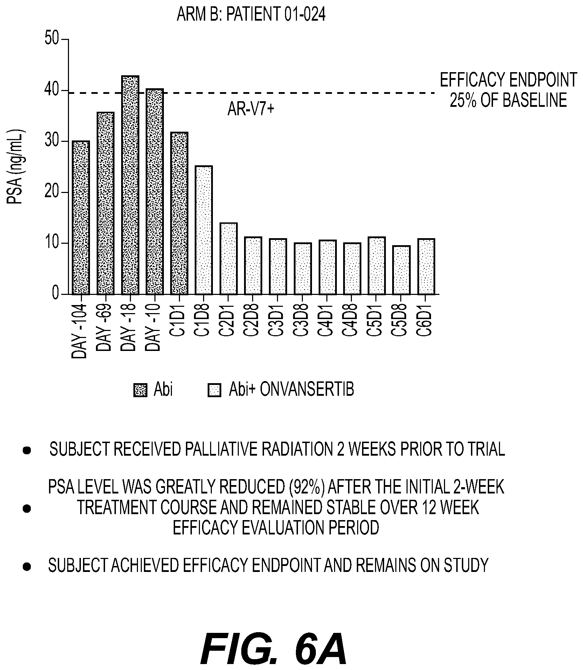

Initial disease stabilization or reduction, based on PSA levels, was achieved in 3 Arm B subjects (FIG. 5). Additionally, disease stabilization after 5 or more treatment cycles was achieved in two patients. One of those patients, 03-013 (FIG. 4) was in Arm A, and the other stabilized patient, 01-024 (FIG. 6A) was in Arm B.

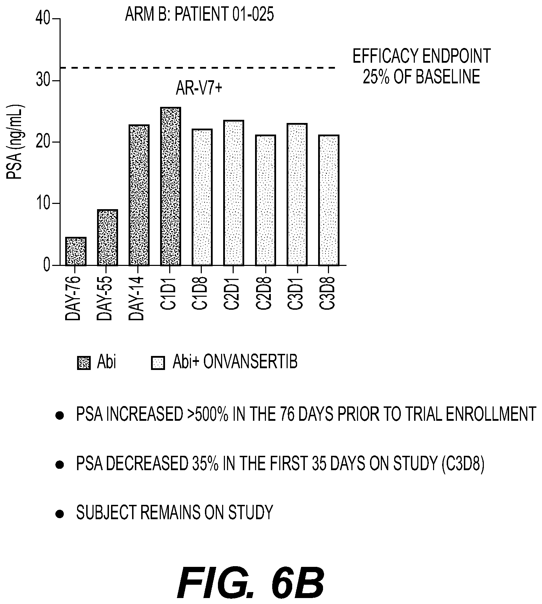

To date (August 2019), initial PSA stabilization or decrease was observed in all AR-V7+ subjects (n=4). Two of these patients met the primary efficacy endpoint: 03-013 (FIG. 4), 01-024 (FIG. 6A), and one patient, 01-025 (FIG. 6B), is still under evaluation.

### Example 4. Further clinical trial results

As of this filing, 63% (12 of 19) patients achieved partial response (PR) or stable disease (SD) following 12 weeks of treatment with onvansertib + abiraterone, based on PSA values (primary endpoint) and radiographic scans.

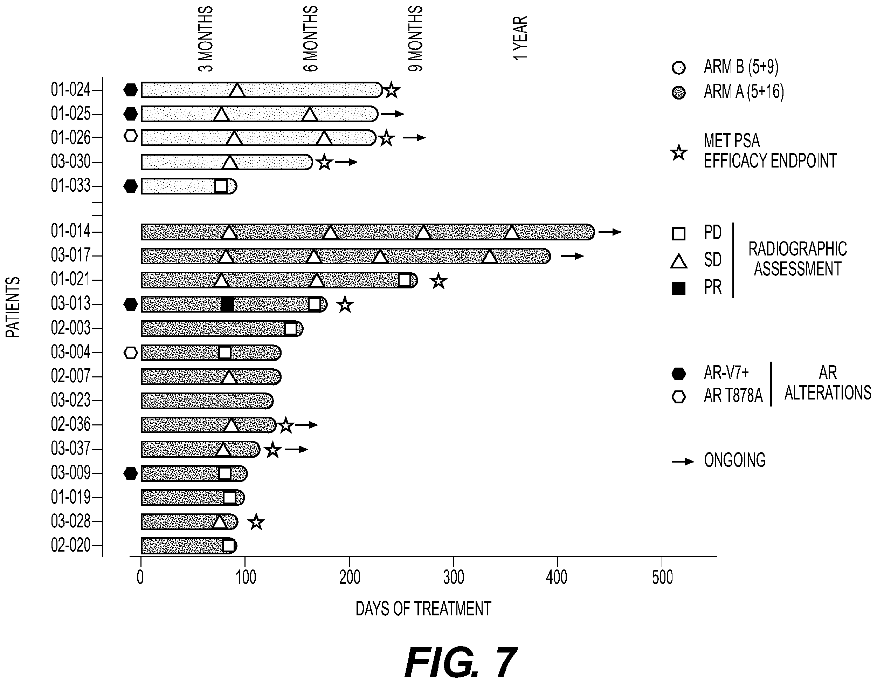

As shown in FIG. 7, in Arm A (n=14), 57% (8 of 14) patients had SD or PR at 12 weeks, with 5 patients achieving the efficacy endpoint (PSA stabilization) and 4 patients remain on treatment; 21% (3 of 14) of Arm A patients have or had progression-free survival; 2 patients remain on treatment for >1 year.

In Arm B (n=5), 80% (4 of 5) patients had SD at 12 weeks, with 3 patients achieving the efficacy endpoint (PSA stabilization) and 3 patients remain on treatment; 60% (3 of 5) of Arm B patients have or had progression-free survival of >7 months.

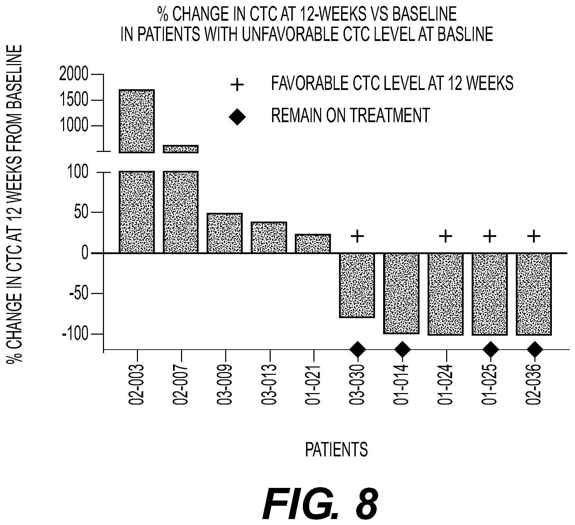

Circulating tumor cell (CTC) evaluation, reported as favorable or unfavorable (<5 vs. ≥CTC/7.5 ml of blood), is shown in FIG. 8. At baseline, 25 (78%) patients had unfavorable CTC count with median of 19 CTC/7.5 ml. Ten of the unfavorable patients were re-analyzed after 12 weeks of treatment. Five (50%) patients had a ≥80% CTC decrease, including 2 AR-V7+ patients (01-024 and 01-025); four (40%) patients converted from unfavorable to favorable CTC level (<5 CTC/7.5 ml); and three (30%) patients had no detectable CTC.

Median time on treatment for patients with decrease CTC (n=5) is 7 months to-date, with 4 patients remaining on treatment. Conversely, median time on treatment for patients with increase CTC (n=5) was 5 months, and none of those patients remain on treatment.

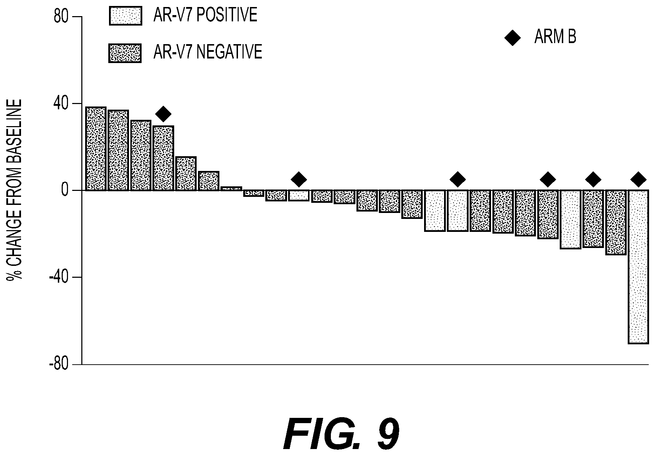

FIG. 9 shows changes in PSA levels with all patients. Eighteen out of 25 (72%) of patients had decreases in PSA levels with the addition of onvansertib after 1 cycle of treatment. Initial PSA stabilization or decrease was observed in all AR-V7 positive patients who completed at least 1 cycle of treatment (n=5). Three of 4 AR-V7 positive patients who have completed 3 months of treatment for efficacy evaluation achieved the primary endpoint of stable disease.

Examples 2-4 establish that the combination of onvansertib and abiraterone offers a new treatment option for patients, including those who are resistant to AR-Signaling Inhibitor (ARSi) therapies, and whose prognosis is poor, including patients having the AR-V7+ androgen receptor.

### References

Armstrong CM, Gao AC. Asian J. Urology. 2019;6:42-49.
Chi K, Annala M, Sunderland K. A randomized phase II cross-over study of abiraterone + prednisone (ABI) vs enzalutamide (ENZ) for patients (pts) with metastatic, castration-resistant prostate cancer (mCRPC). Genitourinary (Prostate) Cancer. Abstract 5002. 2017.
Cao S, et al. Endocr Relat Cancer. 2016;23(12):T199-T210.
de Bono J, Oudard S, Ozguroglu M, et al. Prednisone plus cabazitaxel or mitoxantrone for metastatic castration-resistant prostate cancer progressing after docetaxel treatment: a randomised open-label trial. Lancet. 2010;376(9747):1147-54.
de Bono J, Logothetis C, Molina A, et al. Abiraterone and increased survival in metastatic prostate cancer. N Engl J Med. 2011;364(21):1995-2005.
de Bono J, Spears M. Abiraterone for prostate cancer not previously treated with hormone therapy. N Engl J Med. 2017;377(4):338-351.
Eisenhauer, E, Therasse P, Bogaerts J, et. al. New response evaluation criteria in solid tumours: Revised RECIST guideline (version 1.1). European Journal of Cancer. 2009; 45:228-247.
Fizazi, K, Scher, H, Molina, A, et al. Abiraterone acetate for treatment of metastatic castration-resistant prostate cancer: final overall survival analysis of the COU-AA-301 randomised, double-blind, placebo-controlled phase 3 study. Lancet Oncol. 2012;13(10):983-92.
Fizazi K, Tran N, Fein L. et al. Abiraterone plus Prednisone in Metastatic, Castration-Sensitive Prostate Cancer. N Engl J Med. 2017;377(4):352-360.
Ikezoe T, Yang J, Nishioka C, et al. A novel treatment strategy targeting polo-like kinase 1 in hematological malignancies. Leukemia. 2009; 23(9): 1564-76.
Jackson JR, Patrick DR, Dar MM, Huang PS. Targeted anti-mitotic therapies: can we improve on tubulin agents? Nature Reviews Cancer 2007; 7: 107-117.
Kauselmann G, Weiler M, Wulff P, et al. The polo-like protein kinases Fnk and Snk associate with a Ca (2+)- and integrin-binding protein and are regulated dynamically with synaptic plasticity. EMBO J. 1999; 18(20): 5528-39.
Knecht R, Elez R, Oechler M, et al. Prognostic Significance of Polo-like Kinase (PLK) Expression in Squamous Cell Carcinomas of the Head and Neck. Cancer Res 1999; 59: 2794-2797.
Li J, Karki A, Hodges KB, Cotargeting Polo-Like Kinase 1 and the Wnt/β-Catenin Signaling Pathway in Castration -Resistent Prostate Cancer. Mol Cell Biol 2015 Dec 35(24):4185-8
Li Z, Bishop AC, Alyamani M, et al. Conversion of abiraterone to D4A drives anti-tumour activity in prostate cancer. Nature 2015;523:347-51.
Liu XS, Sonf B, Elzey BD, Polo-Like Kinase 1 Facilitates Loss of PTEN-Induced Prostate Cancer Formation. J Biol Chem 2011 Sep 2;1-12
Mateo J, Carreira S, Sandhu S. DNA-Repair Defects and Olaparib in Metastatic Prostate Cancer. N Engl J Med. 2015 Oct 29;373(18):1697-708.
Mito K, Kashima K, Kikuchi H, et al. Expression of Polo-Like Kinase (PLK1) in non-Hodgkin's lymphomas. Journal: Leukemia and Lymphoma. 2005; 46(2): 225-31.
Parker C, Nilsson S, Heinrich D, et al. Alpha emitter radium-223 and survival in metastatic prostate cancer. N Engl J Med. 2013 Jul 18;369(3):213-23.
Petrylak D, Tangen C, Hussain M, et al. Docetaxel and estramustine compared with mitoxantrone and prednisone for advanced refractory prostate cancer. N Engl J Med. 2004;351(15):1513-20.
Renner AG, Dos Santos C, Recher C, et al. Polo-like kinase 1 is overexpressed in acute myeloid leukemia and its inhibition preferentially targets the proliferation of leukemic cells. Blood. 2009; 114(3): 659-62.
Ryan C, Molina A, Griffin T. Abiraterone in metastatic prostate cancer. N Engl J Med. 2013;368(15):1458-9.
Scher H, Morris M, Stadler W. Trial Design and Objectives for Castration-Resistant Prostate Cancer: Updated Recommendations from the Prostate Cancer Clinical Trials Working Group 3. Journal of Clinical Oncology. 2016;24:1402-18.
Seratec. PSA in body fluids. 2011.
Sharp A, et al. J. Clin. Invest. 2019;129:192-208.
Song MS, Carracedo A, Salmena L, Nuclear PTEN Regulates the APC-CDH1 Tumor-Suppressive Complex in a Phosphatase-Independent Manner. Cell 2011 Jan 21;144:187-199
Strebhardt K, Ullrich A. Targeting polo-like kinase 1 for cancer therapy. Nat Rev Cancer 2006; 6(4):321-30.
Szeliski K et al. Cent European J. Urol. 2018;71:420-426.
Tannock I, de Wit R, Berry W, et al. Docetaxel plus prednisone or mitoxantrone plus prednisone for advanced prostate cancer. N Engl J Med. 2004;351(15):1502-12.
Taplin M, Montgomery B, Logothetis C. Intense androgen-deprivation therapy with abiraterone acetate plus leuprolide acetate in patients with localized high-risk prostate cancer: results of a randomized phase II neoadjuvant study. J Clin Oncol. 2014;32(33):3705-15.
Vlachostergios PJ, et al. Curr Oncol Rep. 2017;19(5):32.
Wadosky KM, Koochekpour S. Oncotarget 2017;8(11):18550-18576.
Weichert W, Schmidt M, Gekeler V, et al. Polo-like kinase 1 is overexpressed in prostate cancer and linked to higher tumor grades. Prostate 2004(a); 60(3): 240-5.
Weichert W, Denkert C, Schmidt M, et al. Polo-like kinase isoform expression is a prognostic factor in ovarian carcinoma. Br. J.Cancer 2004(b); 90: 815-821.
Weichert W, Kristiansen G, Schmidt M, Gekeler V, Noske A, Niesporek S, et al. Polo like kinase 1 expression is a prognostic factor in human colon cancer. World J Gastroenterol. 2005(a); 11(36): 5644-50.
Weichert W, Kristiansen G, Winzer KJ, et al. Polo like kinase isoforms in breast cancer: expression patterns and prognostic implications. Virchows Arch 2005(b); 446: 442-450.
Weiss GJ, Jameson G, VonHoff DD, Phase I dose escalation study of NMS-1286937, an orally available Polo-like Kinase 1 inhibitor, in patients with advanced or metastatic solid tumors. Invest New Drugs. 2017 Jul 20. 10637-017-0491-7.
Wolf G, Elez R, Doermer A, Holtrich U, Ackermann H, Stutte HJ, et al. Prognostic significance of polo-like kinase (PLK) expression in non-small cell lung cancer. Oncogene 1997;14(5): 543-9.
World Health Organization. WHO World Cancer Report. 2014.
Yamaoka M, Hara T, Kusaka M. Overcoming persistent dependency on androgen signaling after progression to castration-resistant prostate cancer. Clin Cancer Res. 2010 Sep 1;16(17):4319-24.
Zhang Z, Hou X, Shao C, et al. PLK-1 inhibition enhances the efficacy of androgen signaling blockade in castration-resistant prostate cancer. Cancer Res. 2014;74(22):6635-47.
Zhang Z, Chen L, Wang H, et al. Inhibition of Plk1 represses androgen signaling pathway in castration-resistant prostate cancer. Cell Cycle. 2015;14(13):2142-8.

In view of the above, it will be seen that several objectives of the invention are achieved and other advantages attained.

As various changes could be made in the above methods and compositions without departing from the scope of the invention, it is intended that all matter contained in the above description and shown in the accompanying drawings shall be interpreted as illustrative and not in a limiting sense.

As used herein, in particular embodiments, the terms "about" or "approximately" when preceding a numerical value indicates the value plus or minus a range of 10%. Where a range of values is provided, it is understood that each intervening value, to the tenth of the unit of the lower limit unless the context clearly dictates otherwise, between the upper and lower limit of that range and any other stated or intervening value in that stated range is encompassed within the disclosure. That the upper and lower limits of these smaller ranges can independently be included in the smaller ranges is also encompassed within the disclosure, subject to any specifically excluded limit in the stated range. Where the stated range includes one or both of the limits, ranges excluding either or both of those included limits are also included in the disclosure.

The indefinite articles "a" and "an," as used herein in the specification and in the embodiments, unless clearly indicated to the contrary, should be understood to mean "at least one."

The phrase "and/or," as used herein in the specification and in the embodiments, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements can optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the embodiments, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the embodiments, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of." "Consisting essentially of," when used in the embodiments, shall have its ordinary meaning as used in the field of patent law.

As used herein in the specification and in the embodiments, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements can optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements).

## Claims

1. Onvansertib for use in a method of treating metastatic castration resistant prostate cancer (mCRPC) in a patient, wherein the patient has mCRPC having an altered androgen receptor AR-V7, the method comprising treating the patient with onvansertib in combination with abiraterone and prednisone.

2. The onvansertib for use of claim 1, wherein the patient also has rising prostate specific antigen (PSA) levels.

3. The onvansertib for use of claim 1, wherein the patient is being treated with abiraterone and prednisone.

## Patentansprüche

1. Onvansertib zur Verwendung in einem Verfahren zur Behandlung von metastasiertem kastrationsresistentem Prostatakrebs (mCRPC) bei einem Patienten, wobei der Patient an mCRPC mit einem veränderten Androgenrezeptor AR-V7 leidet, wobei das Verfahren die Behandlung des Patienten mit Onvansertib in Kombination mit Abirateron und Prednison umfasst.

2. Onvansertib zur Verwendung nach Anspruch 1, wobei der Patient zudem steigende Werte des prostataspezifischen Antigens (PSA) aufweist.

3. Onvansertib zur Verwendung nach Anspruch 1, wobei der Patient mit Abirateron und Prednison behandelt wird.

## Revendications

1. Onvansertib pour utilisation dans une méthode de traitement de cancer de la prostate métastatique résistant à la castration (*metastatic Castration Resistant Prostate Cancer,* mCRPC) chez un patient, dans lequel le patient présente un mCRPC ayant un récepteur d'androgène variant AR-V7, la méthode comprenant le traitement du patient avec l'onvansertib en association avec de l'abiratérone et du prednisone.

2. Onvansertib pour utilisation de la revendication 1, dans lequel le patient présente également des niveaux croissant d'antigène spécifique de la prostate (*Prostate Specific Antigen,* PSA).

3. Onvansertib pour utilisation de la revendication 1, dans lequel le patient est traité avec de l'abiratérone et du prednisone.
